# EUROPEAN PATENT SPECIFICATION

(11) **EP 4 166 070 B1**
(45) Date of publication and mention of the grant of the patent: **08.10.2025**
(21) Application number: 22176109.1
(22) Date of filing: 30.05.2022
(51) Int. Cl.: A61B 5/00, A61B 5/021, A61B 5/024

(54) **METHOD OF EVALUATING QUALITY OF BIO-SIGNAL AND APPARATUS FOR ESTIMATING BIO-INFORMATION**
VERFAHREN ZUM BEWERTEN DER QUALITÄT EINES BIO-SIGNALS UND VORRICHTUNG ZUM SCHÄTZEN VON BIO-INFORMATIONEN
PROCÉDÉ D'ÉVALUATION DE LA QUALITÉ DE BIOSIGNAL ET APPAREIL D'ESTIMATION D'INFORMATIONS BIOLOGIQUES

(30) Priority: 14.10.2021 KR 20210136593
(43) Date of publication of application: 19.04.2023
(73) Proprietor: Samsung Electronics Co., Ltd., Suwon-si, Gyeonggi-do 16677 (KR)
(72) Inventor: Kwon, Ui Kun, Hwaseong-si (KR); Jang, Dae Geun, Yongin-si (KR); Park, Chang Soon, Chungju-si (KR); Kim, Young Soo, Seoul (KR)
(74) Representative: Grünecker Patent- und Rechtsanwälte PartG mbB

(56) References cited:
- EP-A1- 3 492 003
- WO-A1-2018/137300

## Description

### BACKGROUND

### 1. Field

The disclosure relates to estimating bio information, and in particular to a method of evaluating the quality of a bio-signal, and an apparatus for estimating bio-information based on the quality evaluation.

### 2. Description of the Related Art

Due to the aging population, soaring medical costs, and a lack of medical personnel for specialized medical services, research is being actively conducted on IT-medical convergence technologies, in which IT technology and medical technology are combined. Particularly, monitoring of the health condition of the human body is not limited to medical institutions, but is expanding to mobile healthcare fields that may monitor a user's health condition anywhere and anytime in daily life at home or office. Typical examples of biosignals, indicating the health condition of individuals, include an electrocardiography (ECG) signal, a photoplethysmogram (PPG) signal, an electromyography (EMG) signal, etc., and various bio-signal sensors have been developed to measure these signals in daily life. Particularly, a PPG sensor may estimate blood pressure of a human body by analyzing a shape of pulse waves which reflect cardiovascular status and the like.

According to studies on the PPG signal, the whole PPG signal is a summation of a propagation wave propagating from the heart to peripheral parts of a body and reflection waves returning from the peripheral parts of the body. Information for use in estimating blood pressure can be acquired by extracting various features related to propagation waves or reflection waves. However, if the quality of a bio-signal is degraded due to arrhythmia or motion noise in heartbeat, the accuracy in estimating blood pressure may be reduced.
EP 3,492,003 A1 discloses a bio-signal quality assessment apparatus and bio-signal quality assessment method. The bio-signal quality assessment apparatus includes a bio-signal obtainer configured to obtain a bio-signal; and a processor configured to extract periodic signals from the obtained bio-signal, and determine a signal quality index based on at least one of similarity between the extracted periodic signals and signal variability of the obtained bio-signal.
WO 2018/137300 A1 discloses a method and apparatus for determining quality of physiological signal. The method and apparatus for determining quality of a physiological signal relate to the field of wearable devices and is capable of providing the accuracy of the result of determining the quality of the physiological signal and reducing the calculated amount in the process of determining the quality of the physiological signal. The method and apparatus are used in a process of determining the quality of physiological signals before obtaining human body physiological information according to the physiological signals of human body.

### SUMMARY

According to an aspect of the disclosure, a method of evaluating quality of a bio-signal may include: receiving an input bio-signal; setting a quality evaluation region in the bio-signal; dividing a signal of the quality evaluation region into a plurality of sub-signals; extracting a representative waveform by using the plurality of sub-signals; evaluating a quality of each sub-signal based on the representative waveform; and evaluating a quality of the signal in the quality evaluation region based on quality evaluation results of the plurality of sub-signals.

The invention is claimed in the independent claims. Preferred embodiments are specified in the dependent claims.

The setting of the quality evaluation region may include setting the quality evaluation region based on units of a predetermined number of heartbeats or a predetermined time period.

The dividing into the plurality of sub-signals may include dividing the signal of the quality evaluation region into respective sub-signals based on units of beats.

The dividing into the plurality sub-signals may include setting a reference point in the each of the plurality of sub-signals. The extracting of the representative waveform may include extracting the representative waveform, which represents the quality evaluation region, by overlapping the plurality of sub-signals based on the set reference points.

The setting of the reference point may include setting, as the reference point, one of a minimum point, a maximum point, a maximum slope point, and a tangent intersection point of a respective sub-signals.

The extracting of the representative waveform may include overlapping the plurality of sub-signals after applying a predefined weight to each of the plurality of sub-signals.

The extracting of the representative waveform may include adjusting weights to be applied in a current iteration based on quality evaluation results of respective sub-signals in a previous iteration.

The extracting of the representative waveform may include overlapping the plurality of sub-signals after normalizing the plurality sub-signals to a same size.

The evaluating of the quality of each of the plurality of sub-signals may include calculating a similarity between the representative waveform and each sub-signal, or a similarity between an Nth derivative signal of the representative waveform and Nth derivative signals of each sub-signal, and evaluating the quality of each sub-signal based on the calculated similarity.

The evaluating of the quality of each of the plurality of sub-signals may include calculating a similarity between the representative waveform and each of the plurality of sub-signals during a predetermined time interval from the reference point of the each of the plurality of sub-signals.

The extracting of the representative waveform and the evaluating of the quality of each of the plurality of sub-signals may include repeatedly evaluating the quality at least a predetermined number of times and until a similarity between a representative waveform in a current iteration and a representative waveform in a previous iteration are greater than or equal to a first predetermined threshold value, or until quality evaluation results of the plurality of sub-signals in the current iteration and quality evaluation results of the plurality of sub-signals in the previous iteration are greater than or equal to a second predetermined threshold value.

The evaluating of the quality of the signal in the quality evaluation region may include calculating a statistical value of the quality evaluation results of each of the plurality of sub-signals, and evaluating the quality of the signal in the quality evaluation region based on the calculated statistical value.

According to another aspect of the disclosure, an apparatus for estimating bio-information may include a sensor configured to measure a bio-signal from an object; and a processor configured to: set a quality evaluation region in the measured bio-signal; divide a signal of the quality evaluation region into a plurality of sub-signals; extract a representative waveform by using the plurality of sub-signals; evaluate a quality of each of the plurality of sub-signals based on the extracted representative waveform to evaluate a quality of the signal in the quality evaluation region; and estimate bio-information based on the quality evaluation.

The processor may be further configured to set the quality evaluation region based on units of a predetermined number of heartbeats or a predetermined time period.

The processor may be further configured to divide the signal of the quality evaluation region into the plurality of sub-signals based on units of beats.

The processor may be further configured to set a reference point in each of the plurality of sub-signals, and extract the representative waveform, which represents the quality evaluation region, by overlapping the plurality of sub-signals based on the set reference point.

The processor may be further configured to calculate a similarity between the representative waveform and each of the plurality of sub-signals, or a similarity between an Nth derivative signal of the representative waveform and Nth derivative signals of each of the plurality of sub-signals, and evaluate the quality of each of the plurality of sub-signals based on the calculated similarity.

The processor may be further configured to repeatedly evaluate the quality at least a predetermined number of times and until a similarity between a representative waveform in a current iteration and a representative waveform in a previous iteration are greater than or equal to a first predetermined threshold value, or until quality evaluation results of the plurality of sub-signals in the current iteration and quality evaluation results of the plurality of sub-signals in the previous iteration are greater than or equal to a second predetermined threshold value.

The processor may be further configured to calculate a statistical value of the quality evaluation results of each of the plurality of sub-signals, and evaluate the quality of the signal in the quality evaluation region based on the calculated statistical value.

According to yet another aspect of the disclosure, an electronic device may include a main body and an apparatus for estimating blood pressure which is disposed in the main body. The apparatus for estimating blood pressure may include a PPG sensor configured to measure a photoplethysmography (PPG) signal from an object; and a processor configured to: set a quality evaluation region in the PPG signal; divide a signal of the quality evaluation region into a plurality of sub-signals; extract a representative waveform by using the plurality of sub-signals; evaluate a quality of each of the plurality of sub-signals based on the extracted representative waveform to evaluate a quality of the signal in the quality evaluation region; and estimate blood pressure based on the quality evaluation.

According to yet another aspect of the disclosure, a method of evaluating quality of a bio-signal may include receiving a bio-signal based on a first measurement; dividing the bio-signal into a plurality of sub-signals; generating a representative waveform by overlapping the plurality of sub-signals; evaluating a quality of the measured bio-signal by comparing the plurality of sub-signals to the representative waveform; based on a quality of the measured bio-signal being above a predetermined threshold, estimating bio-information based on the measured bio-signal; and based on a quality of the measured bio-signal being below or equal to the predetermined threshold, performing a second measurement for a duration greater than the first measurement and performing a quality evaluation on the second measurement.

### BRIEF DESCRIPTION OF THE DRAWINGS

FIG. 1 is a block diagram of an apparatus for estimating bio-information according to an embodiment.
FIG. 2 is a flowchart of a method of evaluating quality of a bio-signal according to an embodiment.
FIGS. 3A to 6B are diagrams explaining processes of evaluating quality of a bio-signal according to embodiments.
FIG. 7 is a flowchart of a method of evaluating quality of a bio-signal according to an embodiment.
FIG. 8 is a block diagram showing an apparatus for estimating bio-information according to an embodiment.
FIG. 9 is a flowchart of a method of estimating bio-information according to an embodiment.
FIG. 10 is a flowchart of a method of estimating bio-information according to an embodiment.
FIG. 11 is a diagram of a wrist wearable electronic device for estimating bio-information according to an embodiment.
FIG. 12 is a diagram of a mobile electronic device for estimating bio-information according to an embodiment.
FIG. 13 is a diagram of an ear wearable electronic device for estimating bio-information according to an embodiment.

Throughout the drawings and the detailed description, unless otherwise described, the same drawing reference numerals will be understood to refer to the same elements, features, and structures. The relative size and depiction of these elements may be exaggerated for clarity, illustration, and convenience.

### DETAILED DESCRIPTION

Details of example embodiments are included in the following detailed description and drawings. Advantages and features of the present disclosure, and a method of achieving the same will be more clearly understood from the following example embodiments described in detail with reference to the accompanying drawings. Throughout the drawings and the detailed description, unless otherwise described, the same drawing reference numerals will be understood to refer to the same elements, features, and structures.

It will be understood that, although the terms first, second, etc. may be used herein to describe various elements, these elements should not be limited by these terms. These terms are only used to distinguish one element from another. Also, the singular forms are intended to include the plural forms as well, unless the context clearly indicates otherwise. In the specification, unless explicitly described to the contrary, the words "comprise" and "include" and variations such as "comprises," "comprising," "includes," or "including" will be understood to imply the inclusion of stated elements but not the exclusion of any other elements. Terms such as "unit" and "module" denote units that process at least one function or operation, and they may be implemented by using hardware, software, or a combination of hardware and software.

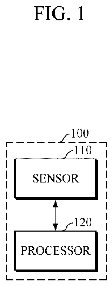

FIG. 1 is a block diagram showing an apparatus for estimating bio-information according to an embodiment.

Referring to FIG. 1, an apparatus 100 for estimating bio-information includes a sensor 110 and a processor 120 (or more than one processor). The sensor 110 and the processor 120 may be integrally formed with each other in a single hardware device, or may be separately formed in two or more hardware devices.

The sensor 110 may acquire a bio-signal having periodicity, i.e., bio-signal having a plurality of repetitive pulse waveforms, by continuously measuring the bio-signal from an object for a predetermined period of time. In this case, the bio-signal may be, for example, electrocardiography (ECG), photoplethysmogram (PPG), ballistocardiogram (BCG), Electromyography (EMG), impedance plethysmogram (IPG), pressure wave, and video plethysmogram (VPG), etc., but is not limited thereto.

For example, the sensor 110 may include a PPG sensor for acquiring a PPG signal from an object, and the PPG sensor may include one or more light sources for emitting light onto a user's object and one or more detectors for detecting light reflected or scattered from the object. The light source may include a light emitting diode (LED), a laser diode (LD), a phosphor, etc., and may be formed as a single light source or an array of two or more light sources. The respective light sources may emit light of different wavelengths. Further, the detector may include a photodiode, a phototransistor, a complementary metal-oxide semiconductor (CMOS) image sensor, a charge-coupled device (CCD) image sensor, etc., and may be formed as a single detector or an array of two or more detectors.

The processor 120 may control the sensor 110, and evaluates the quality of the bio-signal received from the sensor 110. The processor 120 may evaluate the quality of the bio-signal, measured by the sensor 110, by using the received bio-signal itself, a bio-signal filtered by a low-pass filter, a high-pass filter, a band-pass filter, etc., or a bio-signal obtained by Nth order differentiation/integration of the received bio-signal, and the like.

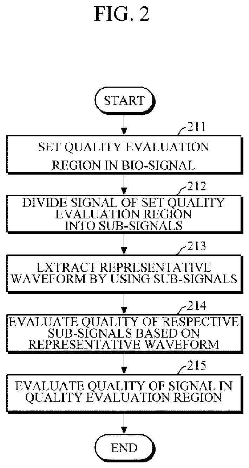

Hereinafter, various examples of determining the quality of the bio-signal will be described with reference to FIGS. 2 to 7 according to embodiment.

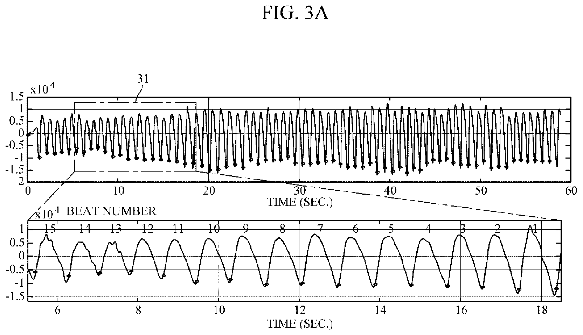

FIG. 2 is a flowchart of a method of evaluating quality of a bio-signal according to an embodiment. FIGS. 3A to 6B are diagrams explaining each process of evaluating quality of a bio-signal according to embodiments.

Referring to FIG. 2, the processor 120 sets a quality evaluation region in a bio-signal, in which quality evaluation is to be performed, in operation 211. FIG. 3A shows a quality evaluation region 31 which is set in units of 15 heartbeats, and the quality evaluation region 31 may be set in units of a predetermined number of heartbeats or a predetermined time period (e.g., a period of 15 seconds).

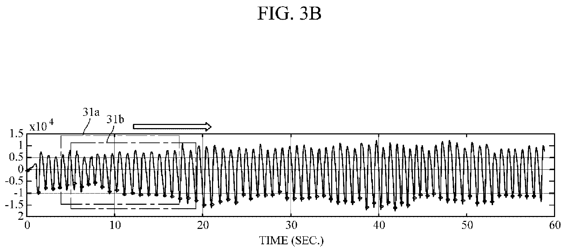

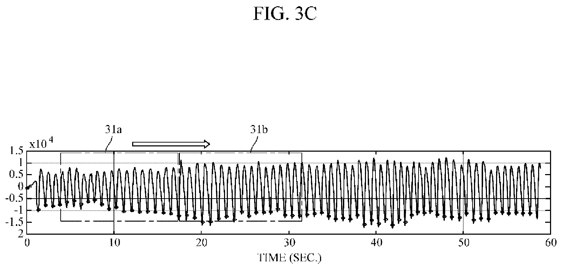

When a continuous bio-signal, measured by the sensor 110, is input in real time, or when input of the measured bio-signal is complete, the processor 120 may check beat counting or elapsed time starting from a predetermined time point, e.g., a measurement start point or a point after a predetermined time elapses, and when a set number of beats is reached or a set time elapses, the processor 120 may set a region from the predetermined time point to a current time point as the quality evaluation region. The quality evaluation region may be set by sliding over time, and operations 212 through 215 which will be described below may be performed for each set quality evaluation region. In this case, quality evaluation regions 31a and 31b may slide to overlap each other as shown in FIG. 3B, or may slide so as not to overlap each other as shown in FIG. 3C.

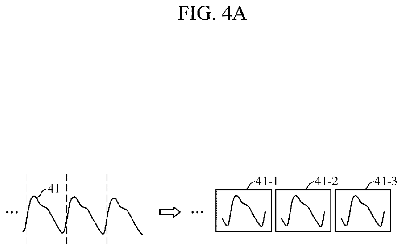

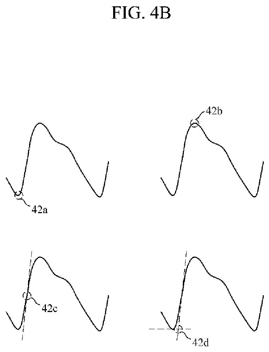

Then, the processor 120 divides a signal of the set quality evaluation region into a plurality of sub-signals in operation 212. For example, as shown in FIG. 4A, the processor 120 may divide the signal 41 of the quality evaluation region into sub-signals 41-1, 41-2, and 41-3. In addition, the processor 120 may set a reference point in each of the divided sub-signals. For example, as shown in FIG. 4B, the processor 120 may set, as the reference point, one of an initial minimum point 42a, a maximum point 42b, a maximum slope point 42c, a tangent intersection point 42d which is an intersection point of a tangent line at the initial minimum point and a tangent line at the maximum slope point, and the like, but the reference point is not limited thereto.

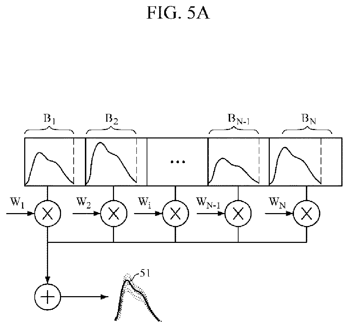

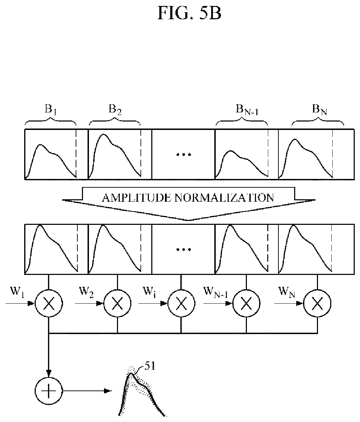

Subsequently, the processor 120 extracts a representative waveform by using the divided sub-signals in operation 213. For example, the processor 120 may extract one representative waveform, which represents the quality evaluation region, by overlapping the respective sub-signals based on the reference points which are set for the respective sub-signals in 212. Referring to FIG. 5A, the processor 120 obtains the representative waveform 51 by applying weights W₁, W₂, W_{N-1}, and W_{N}, which are predetermined for the respective sub-signals B₁, B₂, B _{N-1}, and B_{N}, to the respective sub-signals B₁, B₂, B _{N-1}, and B_{N}. In this case, the weights may be a value equally defined for the respective sub-signals, or may be values including at least some values defined differently. Referring to FIG. 5B, the processor 120 may normalize amplitudes of the respective sub-signals B₁, B₂, B _{N-1}, and B_{N} to the same size, and may obtain the representative waveform 51 by applying weights to the normalized sub-signals B₁, B₂, B _{N-1}, and B_{N}, and then overlapping the sub-signals. In this manner, if an abnormally large noise signal is detected as a sub-signal, the effect of a corresponding noise beat may be reduced.

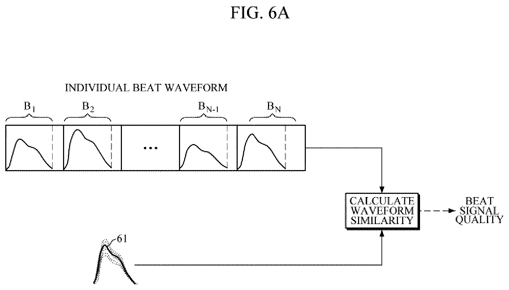

Next, the processor 120 evaluates the quality of the respective sub-signals in operation 214 by using the representative waveform extracted in operation 213. For example, as shown in FIG. 6A, the processor 120 may calculate a similarity between a representative waveform 61 and the respective sub-signals B₁, B₂, B _{N-1}, and B_{N} or a similarity between an Nth derivative signal of the representative waveform 61 and Nth derivative signals of the respective sub-signals B₁, B₂, B _{N-1}, and B_{N}, and may evaluate the quality of the respective sub-signals B₁, B₂, B _{N-1}, and B_{N} by using the calculated similarity. Here, the Nth derivative signal may be a first derivative signal or a second derivative signal, but is not limited thereto. The processor 120 may obtain the similarity value itself or a value, obtained by using a predetermined model, as quality values of the respective sub-signals. In this case, the similarity may be calculated by various methods, such as correlation coefficient, Mean Square Error (MSE), Root Mean Square Error (RMSE), and the like.

That is, the similarity evaluation in this embodiment includes all of the following: similarity evaluation based on an original signal and similarity evaluation based on an Nth derivative signal such as a first derivative signal or a second derivative signal, and it should be understood that unless specifically distinguished, the similarity evaluation includes not only the similarity based on the original signal, but also the similarity based on the Nth derivative signal.

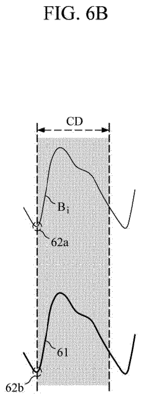

In the case where the sub-signals are divided in units of heartbeats, the respective sub-signals may have different durations due to different heartbeats, and as a result, latter parts of each sub-signal waveform may have different shapes. Accordingly, the processor 120 may calculate a similarity between the representative waveform and the respective sub-signals during a predetermined period of the sub-signals. For example, referring to FIG. 6B, instead of normalizing a length of the representative waveform 61 and the sub-signal Bᵢ to the same size, the processor 120 may calculate a similarity between the representative waveform and a waveform in a predetermined region CD except the latter part of the waveform. For example, the processor 120 may determine, as the similarity calculation region, an interval corresponding to 10 % to 70 % of a duration of the representative waveform, an interval corresponding to 75 % of an average or a median value of durations of the sub-signals, and the like.

Then, the processor 120 evaluates the quality of the signal in the quality evaluation region based on the quality evaluation results of the respective sub-signals in 215. For example, the processor 120 may calculate a statistical value, such as an average, standard deviation, variance, coefficient of variation, etc., of quality values of the respective sub-signals, and may evaluate the quality of the signal in the quality evaluation region based on the calculated statistical value. For example, the processor 120 may determine the calculated statistical value itself or a value, obtained by applying a predefined model, as the signal quality value in the corresponding quality evaluation region. Further, if the signal quality value in the quality evaluation region exceeds a predefined reference value, the processor 120 may determine that the quality is good, and if not, the processor 120 may determine that the quality is bad. Upon completing quality evaluation of the signal in the current quality evaluation region, the processor 120 may proceed to operation 711 for setting a quality evaluation region to evaluate the quality of a signal in a subsequent quality evaluation region.

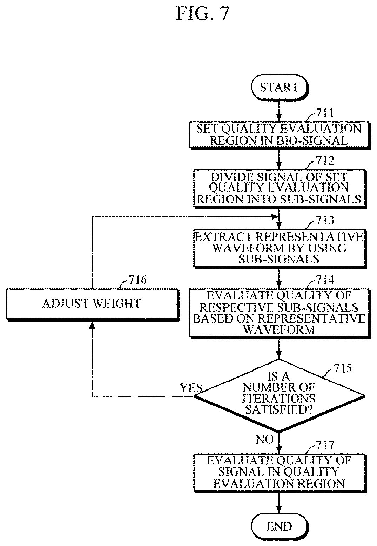

FIG. 7 is a flowchart of a method of evaluating quality of a bio-signal according to an embodiment.

Referring to FIG. 7, the processor 120 may set a quality evaluation region in a bio-signal, in which quality evaluation is to be performed, in operation 711. The quality evaluation region may be set in units of a predetermined number of heartbeats or in units of a predetermined time period. When the bio-signal is input in real time or when input of the measured bio-signal is complete, the processor 120 may check beat counting or elapsed time starting from a predetermined time point, e.g., a measurement start point or a point after a predetermined time elapses, and when a set number of beats is reached or a set time elapses, the processor 120 may set a region from the predetermined time point to a current time point as the quality evaluation region.

Then, the processor 120 may divide a signal of the set quality evaluation region into a plurality of sub-signals in operation 712. The processor 120 may divide the signal of the quality evaluation region into sub-signals in units of beats, and may set a reference point in each of the divided sub-signals.

Subsequently, the processor 120 may extract a representative waveform by using the divided sub-signals in operation 713. In this case, the processor 120 may extract one representative waveform by overlapping the respective sub-signals based on the reference points set for the respective sub-signals, and may overlap the sub-signals by first applying predefined weights, e.g., a fixed value (e.g., 1) equally defined for the respective sub-signals, or values including at least some values defined differently, to the respective sub-signals. In addition, the processor 120 may overlap the respective sub-signals after normalizing amplitudes of the respective sub-signals to the same size.

Next, the processor 120 may evaluate the quality of the respective sub-signals in operation 714 by using the representative waveform extracted in 713. For example, the processor 120 may calculate a similarity between a representative waveform and the respective sub-signals, and may evaluate the quality of the respective sub-signals based on the calculated similarity. For example, the processor 120 may obtain the similarity value itself of the respective sub-signals or a value, obtained by using a predetermined model, as the quality value of the respective sub-signals. In this case, the processor 120 may calculate the similarity between the representative waveform and the respective sub-signals during a predetermined period of the sub-signals.

Then, the processor 120 may determine whether a number of iterations for extracting the representative waveform is satisfied in operation 715. For example, if a number of a current iteration is less than a predefined reference value, the processor 120 may determine that a next iteration for extracting a representative waveform is to be performed. In another example, the processor 120 may calculate a similarity between the representative waveform extracted in the current iteration and a representative waveform extracted in a previous iteration, and if the calculated similarity is less than a predetermined threshold value, the processor 120 may determine to perform the next iteration for extracting a representative waveform. In yet another example, if a difference between quality evaluation results of the respective sub-signals in the current iteration and quality evaluation results of the respective sub-signals in the previous iteration, e.g., a difference between a statistical value (e.g., average, median value, etc.) of the quality evaluation results of the sub-signals in the current iteration and a statistical value of the quality evaluation results of the sub-signals in the previous iteration exceeds a predetermined threshold value, the processor 120 may determine that the next iteration is to be performed. However, the determination is not limited thereto.

Subsequently, upon determining in operation 715 that the next iteration is to be performed, the processor 120 adjusts a weight to be applied to the respective sub-signals in the next iteration in operation 716, and may perform operations 713 and 714 again. For example, based on the quality evaluation results of the respective sub-signals which are calculated in the current iteration of operation 714, the processor 120 adjusts the weight for the respective sub-signals. The processor 120 sets quality values of the respective sub-signals in the current iteration as weights for the respective sub-signals.

Next, based on the quality evaluation results of the respective sub-signals, the processor 120 may evaluate the quality of the signal in the quality evaluation region in operation 717. For example, the processor 120 may calculate a statistical value, such as an average, standard deviation, variance, coefficient of variation, etc., of the quality values of the respective sub-signals, and may evaluate the quality of the signal in the quality evaluation region based on the calculated statistical value. For example, the processor 120 may determine the calculated statistical value itself or a value, obtained by applying a predefined model, as the signal quality value in the corresponding quality evaluation region. Further, if the signal quality value in the quality evaluation region exceeds a predefined threshold value, the processor 120 may determine that the quality is good, and if not, the processor 120 may determine that the quality is bad.

Referring back to FIG. 1, the processor 120 may estimate bio-information based on the quality evaluation result of the bio-signal. In this case, the bio-information may include blood pressure, arrhythmia, vascular age, skin elasticity, skin age, arterial stiffness, aortic pressure waveform, stress index, fatigue level, etc., but is not limited thereto.

For example, the processor 120 may estimate bio-information by using a signal in the quality evaluation region in which the quality evaluation result is good. In this case, if there are a plurality of quality evaluation regions having good quality evaluation results, the processor 120 may select, for example, any one quality evaluation region in order of the quality values of the respective quality evaluation regions, or may select two or more quality evaluation regions and may use signals of the respective equality evaluation regions.

The processor 120 may extract one or more features from the representative waveform extracted from the corresponding quality evaluation region. For example, the processor 120 may extract, as features, amplitude and/or time values associated with propagation waves and reflection waves, shape of a waveform, time and/or amplitude values at a maximum point during a systolic phase of the waveform, time and/or amplitude values at a minimum point, a total or partial area of the waveform, elapsed time, and the like. However, these are merely examples.

The processor 120 may estimate bio-information by combining one or two or more of the extracted features and by using a predefined bio-information estimation model. The bio-information estimation model may be predefined by using various methods, such as a linear function equation, nonlinear regression analysis, neural network, deep learning, and the like.

In another example, if there is no quality evaluation region of the input bio-signal in which a quality evaluation result is good, the processor 120 may guide a user to re-measure a bio-signal, or may terminate estimating the bio-signal.

In yet another example, by controlling the sensor 110 to measure the bio-signal for a first period of time (e.g., 40 seconds), the processor 120 may evaluate the quality of the signal input from the sensor 110, and if there is no quality evaluation region having a good signal quality, the processor 120 may increase the measurement time to control the sensor 110 to further measure the bio-signal continuously for a second period of time (e.g. 20 seconds) following the first period time. Based on the bio-signal measured during the first period of time and/or the second period of time, the processor 620 may perform quality evaluation again.

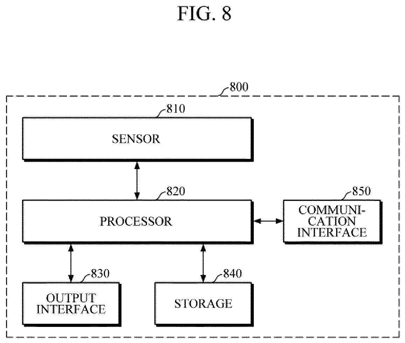

FIG. 8 is a block diagram of an apparatus for estimating bio-information according to an embodiment.

Referring to FIG. 8, an apparatus 800 for estimating bio-information may include a sensor 810, a processor 820, an output interface 830, a storage 840, and a communication interface 850. In this case, various embodiments of the sensor 810 and the processor 820 are described above with reference to FIGS. 1 to 7, such that a description thereof will be omitted.

The output interface 830 may provide processing results of the processor 820 for a user. For example, the output interface 830 may display an estimated bio-information value of the processor 820 on a display. In this case, if the estimated blood pressure value falls outside a normal range, the output interface 830 may provide a user with warning information by changing color, line thickness, etc., or displaying an abnormal value along with a normal range, so that the user may easily recognize the abnormal value. Further, along with or without the visual displaying of the value, the output interface 830 may provide the user with the estimated bio-information value in a non-visual manner by voice, vibrations, tactile sensation, and the like using an audio output module such as a speaker, or a haptic module and the like.

Further, the output interface 830 may display a quality evaluation process performed by the processor 820, a quality evaluation result, and the like in a visual manner such as a graph. In addition, if the quality of the bio-signal is bad based on the quality evaluation result, the output interface 830 may guide a user to re-measure a bio-signal, to additionally measure a bio-signal, or to terminate estimating the bio-signal.

The storage 840 may store information related to estimating bio-information. For example, the storage 840 may store the bio-signal, acquired by the sensor 810, and the processing results of the processor 820, e.g., the quality evaluation result and the estimated bio-information value. Further, the storage 840 may store the bio-information estimation model, units of quality evaluation regions, a threshold value used for quality evaluation, user characteristic information, and the like. In this case, the user characteristic information may include a user's age, gender, health condition, and the like.

The storage 840 may include at least one storage medium of a flash memory type memory, a hard disk type memory, a multimedia card micro type memory, a card type memory (e.g., an SD memory, an XD memory, etc.), a Random Access Memory (RAM), a Static Random Access Memory (SRAM), a Read Only Memory (ROM), an Electrically Erasable Programmable Read Only Memory (EEPROM), a Programmable Read Only Memory (PROM), a magnetic memory, a magnetic disk, and an optical disk, and the like, but is not limited thereto.

The communication interface 850 may communicate with an external device to transmit and receive various data related to estimating bio-information. The external device may include an information processing device such as a smartphone, a tablet PC, a desktop computer, a laptop computer, and the like. For example, the communication interface 850 may transmit a bio-information estimation result to the external device, such as a user's smartphone and the like, so that the user may manage and monitor component analysis results using a device having a relatively high performance. In addition, in the case where the external device includes a sensor for measuring a bio-signal, the communication interface 850 may receive a bio-signal for quality evaluation from the external device.

The communicator 850 may communicate with the external device by using various wired or wireless communication techniques, such as Bluetooth communication, Bluetooth Low Energy (BLE) communication, Near Field Communication (NFC), WLAN communication, Zigbee communication, Infrared Data Association (IrDA) communication, Wi-Fi Direct (WFD) communication, Ultra-Wideband (UWB) communication, Ant+ communication, WIFI communication, Radio Frequency Identification (RFID) communication, 3G, 4G, and 5G communications, and the like. However, this is merely exemplary and is not intended to be limiting.

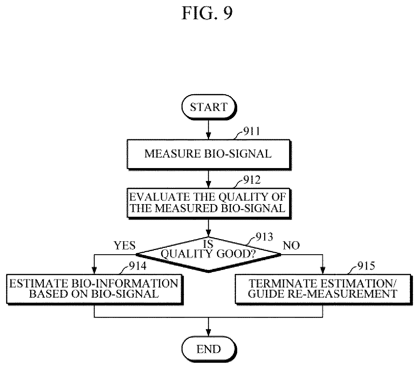

FIG. 9 is a flowchart of a method of estimating bio-information according to an embodiment .

The method of FIG. 9 is an example of a method of estimating bio-information which may be performed by the apparatus 100 or 800 for estimating bio-information according to the embodiment of FIG. 1 or FIG. 8, which will be briefly described below in order to avoid redundancy.

Referring to FIG. 9, the apparatus for estimating bio-information may first measure a bio-signal from a user's object using a sensor in operation 911, and may evaluate the quality of the measured bio-signal in operation 912. A method of evaluating the quality of the bio-signal is described above in detail.

Then, if the quality of the bio-signal is good in operation 913, the apparatus for estimating bio-information may extract one or more features from the good quality signal in a quality evaluation region, and may estimate bio-information by using the extracted features in operation 914. If the quality of the bio-signal is bad in operation 913, the apparatus for estimating bio-information may terminate estimating the bio-information or may guide the user to re-measure the bio-signal in operation 915.

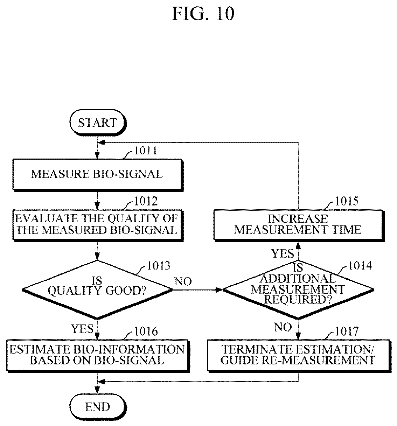

FIG. 10 is a flowchart of a method of estimating bio-information according to an embodiment.

The method of FIG. 10 is an example of a method of estimating bio-information which may be performed by the apparatus 100 or 800 for estimating bio-information according to the embodiment of FIG. 1 or FIG. 8, which will be briefly described below in order to avoid redundancy.

Referring to FIG. 10, the apparatus for estimating bio-information may first measure a bio-signal from a user's object using a sensor in operation 1011, and may evaluate the quality of the measured bio-signal in operation 1012. A method of evaluating the quality of the bio-signal is described above in detail.

Then, if the quality of the bio-signal is bad in operation 1013, the apparatus for estimating bio-information may determine whether to additionally measure a bio-signal in operation 1014, and if additional measurement is required, the apparatus for estimating bio-information may increase a measurement time in operation 1015, and may proceed to operation 1011 to additionally measure a bio-signal continuously for an increased period of time. Upon determining that the additional measurement is not required in operation 1014, the apparatus for estimating bio-information may terminate estimating bio-information or may guide the user to re-measure the bio-signal in operation 1017.

If the quality of the bio-signal is good in operation 1013, the apparatus for estimating bio-information may estimate bio-information by using the bio-signal in operation 1016. In this case, the apparatus for estimating bio-information may extract features from the good quality signal in a quality evaluation region, and may estimate bio-information by using the extracted features and a bio-information estimation model.

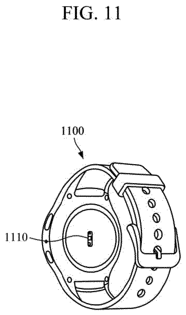

FIGS. 11 to 13 are block diagrams showing various structures of an electronic device including the apparatus 100 or 800 for estimating bio-information of FIG. 1 or FIG. 8 according to embodiments.

The electronic device may include, for example, various types of wearable devices, e.g., a smart watch, a smart band, smart glasses, smart earphones, a smart ring, a smart patch, and a smart necklace, and a mobile device such as a smartphone, a tablet PC, etc., or home appliances or various Internet of Things (IoT) devices (e.g., home IoT device, etc.) based on Internet of Things (IoT) technology.

The electronic device may include a sensor device, a processor, an input device, a communication module, a camera module, an output device, a storage device, and a power module. All the components of the electronic device may be integrally mounted in a specific device or may be distributed in two or more devices. The sensor device may include the sensor (e.g., PPG sensor) of the apparatuses 100 and 800 for estimating bio-information, and may further include an additional sensor, such as a gyro sensor, a Global Positioning System (GPS), and the like.

The processor may execute programs, stored in the storage device, to control components connected to the processor, and may perform various data processing or computation, including estimation of bio-information (e.g., blood pressure). For example, the processor may evaluate the quality of a PPG signal, measured by using the PPG sensor of the sensor device, and may estimate blood pressure based on the evaluation result. Various embodiments of evaluating the quality and estimating blood pressure are described above, such that a detailed description thereof will be omitted. The processor may include a main processor, e.g., a central processing unit (CPU) or an application processor (AP), etc., and an auxiliary processor, e.g., a graphics processing unit (GPU), an image signal processor (ISP), a sensor hub processor, or a communication processor (CP), etc., which is operable independently from, or in conjunction with, the main processor.

The input device may receive a command and/or data to be used by each component of the electronic device, from a user and the like. The input device may include, for example, a microphone, a mouse, a keyboard, or a digital pen (e.g., a stylus pen, etc.).

The communication module may support establishment of a direct (e.g., wired) communication channel and/or a wireless communication channel between the electronic device and other electronic device, a server, or the sensor device within a network environment, and performing of communication via the established communication channel. The communication module may include one or more communication processors that are operable independently from the processor and supports a direct communication and/or a wireless communication. The communication module may include a wireless communication module, e.g., a cellular communication module, a short-range wireless communication module, or a global navigation satellite system (GNSS) communication module, etc., and/or a wired communication module, e.g., a local area network (LAN) communication module, a power line communication (PLC) module, and the like. These various types of communication modules may be integrated into a single chip, or may be separately implemented as multiple chips. The wireless communication module may identify and authenticate the electronic device in a communication network by using subscriber information (e.g., international mobile subscriber identity (IMSI), etc.) stored in a subscriber identification module.

The camera module may capture still images or moving images. The camera module may include a lens assembly having one mor more lenses, image sensors, image signal processors, and/or flashes. The lens assembly included in the camera module may collect light emanating from a subject to be imaged.

The output device may visually/non-visually output data generated or processed by the electronic device. The output device may include a sound output device, a display device, an audio module, and/or a haptic module.

The sound output device may output sound signals to the outside of the electronic device. The sound output device may include a speaker and/or a receiver. The speaker may be used for general purposes, such as playing multimedia or playing record, and the receiver may be used for incoming calls. The receiver may be implemented separately from, or as part of, the speaker.

The display device may visually provide information to the outside of the electronic device. The display device may include, for example, a display, a hologram device, or a projector and control circuitry to control the devices. The display device may include touch circuitry adapted to detect a touch, and/or sensor circuitry (e.g., pressure sensor, etc.) adapted to measure the intensity of force incurred by the touch.

The audio module may convert a sound into an electrical signal or vice versa. The audio module may obtain the sound via the input device, or may output the sound via the sound output device, and/or a speaker and/or a headphone of another electronic device directly or wirelessly connected to the electronic device.

The haptic module may convert an electrical signal into a mechanical stimulus (e.g., vibration, motion, etc.) or electrical stimulus which may be recognized by a user by tactile sensation or kinesthetic sensation. The haptic module may include, for example, a motor, a piezoelectric element, and/or an electric stimulator.

The storage device may store driving conditions required for driving the sensor device, and various data required for other components of the electronic device. The various data may include, for example, software and input data and/or output data for a command related thereto. The storage device may include a volatile memory and/or a non-volatile memory.

The power module may manage power supplied to the electronic device. The power module may be implemented as least part of, for example, a power management integrated circuit (PMIC). The power module may include a battery, which may include a primary cell which is not rechargeable, a secondary cell which is rechargeable, and/or a fuel cell.

Referring to FIG. 11, according to an embodiment, the electronic device may be implemented as a wristwatch wearable device 1100, and may include a main body and a wrist strap. A display is provided on a front surface of the main body, and may display various application screens, including time information, received message information, and the like. A sensor device 1110 may be disposed on a rear surface of the main body.

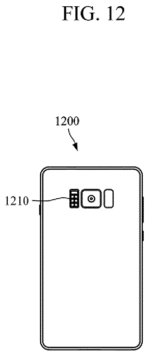

Referring to FIG. 12, according to an embodiment, the electronic device may be implemented as a mobile device 1200 such as a smartphone.

The mobile device 1200 may include a housing and a display panel. The housing may form an exterior of the mobile device 1200. The housing has a first surface, on which a display panel and a cover glass may be disposed sequentially, and the display panel may be exposed to the outside through the cover glass. The sensor device 1110, a camera module and/or an infrared sensor, and the like may be disposed on a second surface of the housing. The processor and various other components may be disposed in the housing.

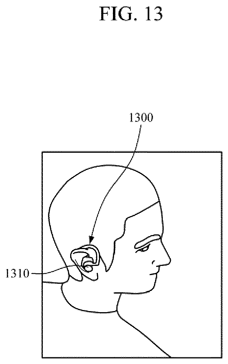

Referring to FIG. 13, according to an embodiment, the electronic device may be implemented as an ear-wearable device 1300.

The ear-wearable device 1300 may include a main body and an ear strap. A user may wear the ear-wearable device 1300 by hanging the ear strap on the auricle. The ear strap may be omitted depending on a shape of the ear-wearable device 1300. The main body may be inserted into the external auditory meatus. A sensor device 1310 may be mounted in the main body. Further, the processor may be disposed in the main body, and may estimate blood pressure by using a pulse wave signal measured by the sensor device 1310. Alternatively, the ear-wearable device 1300 may estimate blood pressure by interworking with an external device. For example, the pulse wave signal, measured by the sensor device 1310 of the ear-wearable device 1300, may be transmitted to an external device, e.g., a mobile device, a tablet PC, etc., through a communication module provided in the main body, so that a processor of the external device may estimate blood pressure, and the estimated blood pressure value may be output through a sound output module provided in the main body of the ear-wearable device 1300.

The present disclosure can be realized as a computer-readable code written on a computer-readable recording medium. The computer-readable recording medium may be any type of recording device in which data is stored in a computer-readable manner.

Examples of the computer-readable recording medium include a ROM, a RAM, a CD-ROM, a magnetic tape, a floppy disc, an optical data storage, and a carrier wave (e.g., data transmission through the Internet). The computer-readable recording medium can be distributed over a plurality of computer systems connected to a network so that a computer-readable code is written thereto and executed therefrom in a decentralized manner. Functional programs, codes, and code segments needed for realizing the present disclosure can be readily deduced by programmers of ordinary skill in the art to which the disclosure pertains.

## Claims

1. A computer-implemented method of evaluating quality of a bio-signal, the method comprising:
receiving a measured bio-signal;
setting (211, 711) a quality evaluation region in the measured bio-signal;
dividing (212, 712) a signal of the quality evaluation region into a plurality of sub-signals (B₁, B₂, ..., B_{N-1}, B_{N});
extracting (213, 713) a representative waveform (41, 51, 61) by using the plurality of divided sub-signals (B₁, B₂, ..., B_{N-1}, B_{N});
evaluating (214, 714) a quality of each sub-signal (B₁, B₂, ..., B_{N-1}, B_{N}) based on the extracted representative waveform;
evaluating (215, 717) a quality of the signal in the quality evaluation region based on quality evaluation results of the plurality of sub-signals; and
estimating bio-information based on the quality evaluation,
wherein the representative waveform (41, 51, 61) is obtained (213, 713) by applying weights (W₁, W₂, ..., W_{N-1}, W_{N}), which are predetermined for the respective sub-signals (B₁, B₂, ..., B_{N-1}, B_{N}), to the respective sub-signals (B₁, B₂, ..., B_{N-1}, B_{N}), and
wherein the extracting (213, 713) of the representative waveform further comprises adjusting (716) the weights to be applied in a current iteration based on quality evaluation results of respective sub-signals in a previous iteration.

2. The method of claim 1, wherein the setting (211, 711) of the quality evaluation region comprises setting the quality evaluation region based on units of a predetermined number of heartbeats or a predetermined time period.

3. The method of claim 1 or 2, wherein the dividing into the plurality of sub-signals comprises one of:
dividing the signal of the quality evaluation region into respective sub-signals based on units of beats;
setting a reference point in the each of the plurality of sub-signals; and
wherein the extracting of the representative waveform comprises extracting the representative waveform, which represents the quality evaluation region, by overlapping the plurality of sub-signals based on the set reference point.

4. The method of claim 3, wherein the setting of the reference point (42a, 42b, 42c, 42d) comprises setting, as the reference point, one of a minimum point (42a), a maximum point (42b), a maximum slope point (42c), and a tangent intersection point (42d) of a respective sub-signals.

5. The method of claim 3, wherein the extracting of the representative waveform comprises overlapping the plurality of sub-signals after applying a predefined weight to each of the plurality of sub-signals.

6. The method of claim 3, wherein the extracting (213, 713) of the representative waveform comprises overlapping the plurality of sub-signals after normalizing the plurality sub-signals (B₁, B₂, ..., B_{N-1}, B_{N}) to a same size.

7. The method of one of claims 1 to 6, wherein the evaluating (214, 714) of the quality of each of the plurality of sub-signals (B₁, B₂, ..., B_{N-1}, B_{N}) comprises calculating a similarity between the representative waveform (41, 51, 61) and each sub-signal (B₁, B₂, ..., B_{N-1}, B_{N}), or a similarity between an Nth derivative signal of the representative waveform (41, 51, 61) and Nth derivative signals of each sub-signal, and evaluating the quality of each sub-signal (B₁, B₂, ..., B_{N-1}, B_{N}) based on the calculated similarity,
wherein the evaluating of the quality of each of the plurality of sub-signals (B₁, B₂, ..., B_{N-1}, B_{N}) comprises calculating a similarity between the representative waveform (41, 51, 61) and each of the plurality of sub-signals (B₁, B₂, ..., B_{N-1}, B_{N}) during a predetermined time interval from the reference point (42a, 42b, 42c, 42d) of the each of the plurality of sub-signals (B₁, B₂, ..., B_{N-1}, B_{N}).

8. The method of one of claims 1 to 7, wherein the extracting (213, 713) of the representative waveform (41, 51, 61) and the evaluating (214, 714) of the quality of each of the plurality of sub-signals (B₁, B₂, ..., B_{N-1}, B_{N}) comprise repeatedly evaluating the quality at least a predetermined number of times and until a similarity between a representative waveform in a current iteration and a representative waveform in a previous iteration are greater than or equal to a first predetermined threshold value, or until quality evaluation results of the plurality of sub-signals in the current iteration and quality evaluation results of the plurality of sub-signals in the previous iteration are greater than or equal to a second predetermined threshold value.

9. The method of one of claims 1 to 8, wherein the evaluating (215, 717) of the quality of the signal in the quality evaluation region comprises calculating a statistical value of the quality evaluation results of each of the plurality of sub-signals, and evaluating the quality of the signal in the quality evaluation region based on the calculated statistical value.

10. An apparatus for estimating bio-information, the apparatus comprising:
a sensor (110, 810) configured to measure a bio-signal from an object; and
a processor (120, 820) configured to:
set (211, 711) a quality evaluation region in the measured bio-signal;
divide (212, 712) a signal of the quality evaluation region into a plurality of sub-signals;
extract (213, 713) a representative waveform (41, 51, 61) by using the plurality of divided sub-signals (B₁, B₂, ..., B_{N-1}, B_{N});
evaluate (214, 714) a quality of each of the plurality of sub-signals (B₁, B₂, ... , B_{N-1}, B_{N}) based on the extracted representative waveform to evaluate (215, 717) a quality of the signal in the quality evaluation region; and
estimate bio-information based on the quality evaluation,
wherein the processor (120, 820) is further configured to obtain (213, 713) the representative waveform (41, 51, 61) by applying weights (W₁, W₂, ..., W_{N-1}, W_{N}), which are predetermined for the respective sub-signals (B₁, B₂, ..., B_{N-1}, B_{N}), to the respective sub-signals (B₁, B₂, ..., B_{N-1}, B_{N}), and to extract (213, 713) the representative waveform (41, 51, 61) by adjusting (716) the weights to be applied in a current iteration based on quality evaluation results of respective sub-signals in a previous iteration.

11. The apparatus of claim 10, wherein the processor (120, 820) is further configured to perform one of:
setting the quality evaluation region based on units of a predetermined number of heartbeats or a predetermined time period;
dividing the signal of the quality evaluation region into the plurality of sub-signals based on units of beats;
setting a reference point in each of the plurality of sub-signals, and extracting the representative waveform, which represents the quality evaluation region, by overlapping the plurality of sub-signals based on the set reference point; and
calculating a similarity between the representative waveform and each of the plurality of sub-signals, or a similarity between an Nth derivative signal of the representative waveform and Nth derivative signals of each of the plurality of sub-signals, and evaluate the quality of each of the plurality of sub-signals based on the calculated similarity.

12. The apparatus of claim 10 or 11, wherein the processor (120, 820) is further configured to repeatedly evaluate the quality at least a predetermined number of times and until a similarity between a representative waveform in a current iteration and a representative waveform in a previous iteration are greater than or equal to a first predetermined threshold value, or until quality evaluation results of the plurality of sub-signals in the current iteration and quality evaluation results of the plurality of sub-signals in the previous iteration are greater than or equal to a second predetermined threshold value.

13. The apparatus of one of claims 10 to 12, wherein the processor (120, 820) is further configured to calculate a statistical value of the quality evaluation results of each of the plurality of sub-signals, and evaluate the quality of the signal in the quality evaluation region based on the calculated statistical value.

14. An electronic device comprising a main body and an apparatus for estimating bio-information according to one of claims 10 to 13 which is disposed in the main body.

15. A computer-readable storage medium storing instructions that, when executed by a processor (120, 820), cause the processor (120, 820) to perform the method of evaluating quality of a bio-signal comprising the steps of:
receiving a measured bio-signal;
setting (211, 711) a quality evaluation region in the measured bio-signal;
dividing (212, 712) a signal of the quality evaluation region into a plurality of sub-signals (B₁, B₂, ..., B_{N-1}, B_{N});
extracting (213, 713) a representative waveform (41, 51, 61) by using the plurality of divided sub-signals (B₁, B₂, ..., B_{N-1}, B_{N});
evaluating (214, 714) a quality of each sub-signal (B₁, B₂, ..., B_{N-1}, B_{N}) based on the extracted representative waveform;
evaluating (215, 717) a quality of the signal in the quality evaluation region based on quality evaluation results of the plurality of sub-signals; and
estimating bio-information based on the quality evaluation,
wherein the processor (120, 820) is further instructed to obtain (213, 713) the representative waveform (41, 51, 61) by applying weights (W₁, W₂, ..., W_{N-1}, W_{N}), which are predetermined for the respective sub-signals (B₁, B₂, ..., B_{N-1}, B_{N}), to the respective sub-signals (B₁, B₂, ..., B_{N-1}, B_{N}), and to extract (213, 713) the representative waveform (41, 51, 61) by adjusting (716) the weights to be applied in a current iteration based on quality evaluation results of respective sub-signals in a previous iteration.

## Patentansprüche

1. Computerimplementiertes Verfahren zum Bewerten der Qualität eines Biosignals, wobei das Verfahren umfasst:
Empfangen eines gemessenen Biosignals;
Festlegen (211, 711) eines Qualitätsbewertungsbereichs im gemessenen Biosignal;
Unterteilen (212, 712) eines Signals des Qualitätsbewertungsbereichs in eine Vielzahl von Teilsignalen (B₁, B₂, ..., B_{N-1}, B_{N});
Extrahieren (213, 713) einer repräsentativen Wellenform (41, 51, 61) unter Verwendung der Vielzahl von unterteilten Teilsignalen (B₁, B₂, ..., B_{N-1}, B_{N});
Bewerten (214, 714) einer Qualität jedes Teilsignals (B₁, B₂, ..., B_{N-1}, B_{N}) basierend auf der extrahierten repräsentativen Wellenform;
Bewerten (215, 717) einer Qualität des Signals im Qualitätsbewertungsbereich basierend auf Qualitätsbewertungsergebnissen der Vielzahl von Teilsignalen; und
Schätzen von Bioinformationen basierend auf der Qualitätsbewertung,
wobei die repräsentative Wellenform (41, 51, 61) durch Anwenden von Gewichten (W₁, W₂, ..., W_{N-1}, W_{N}), die für die jeweiligen Teilsignale (B₁, B₂, ..., B_{N-1}, B_{N}) vorbestimmt sind, auf die jeweiligen Teilsignale (B₁, B₂, ..., B_{N-1}, B_{N}) erhalten wird (213, 713), und
wobei das Extrahieren (213, 713) der repräsentativen Wellenform ferner das Anpassen (716) der in einer aktuellen Iteration anzuwendenden Gewichte basierend auf Qualitätsbewertungsergebnissen jeweiliger Teilsignale in einer vorhergehenden Iteration umfasst.

2. Verfahren nach Anspruch 1, wobei das Festlegen (211, 711) des Qualitätsbewertungsbereichs das Festlegen des Qualitätsbewertungsbereichs basierend auf Einheiten einer vorbestimmten Zahl von Herzschlägen oder eines vorbestimmten Zeitraums umfasst.

3. Verfahren nach Anspruch 1 oder 2, wobei das Unterteilen in die Vielzahl von Teilsignalen eines der folgenden Elemente umfasst:
Unterteilen des Signals des Qualitätsbewertungsbereichs in jeweilige Teilsignale basierend auf Schlageinheiten;
Festlegen eines Referenzpunkts in jedem der Vielzahl von Teilsignalen; und
wobei das Extrahieren der repräsentativen Wellenform das Extrahieren der repräsentativen Wellenform, die den Qualitätsbewertungsbereich darstellt, durch Überlappen der Vielzahl von Teilsignalen basierend auf dem festgelegten Referenzpunkt umfasst.

4. Verfahren nach Anspruch 3, wobei das Festlegen des Referenzpunkts (42a, 42b, 42c, 42d) das Festlegen eines Elements der Gruppe umfassend einen Minimalpunkt (42a), einen Maximalpunkt (42b), einen Punkt maximaler Neigung (42c) und einen Tangentenschnittpunkt (42d) eines jeweiligen Teilsignals als Referenzpunkt umfasst.

5. Verfahren nach Anspruch 3, wobei das Extrahieren der repräsentativen Wellenform das Überlappen der Vielzahl von Teilsignalen nach Anwenden eines vordefinierten Gewichts auf jedes der Vielzahl von Teilsignalen umfasst.

6. Verfahren nach Anspruch 3, wobei das Extrahieren (213, 713) der repräsentativen Wellenform das Überlappen der Vielzahl von Teilsignalen nach dem Normalisieren der Vielzahl von Teilsignalen (B₁, B₂, ..., B_{N-1}, B_{N}) zu einer gleichen Größe umfasst.

7. Verfahren nach einem der Ansprüche 1 bis 6, wobei das Bewerten (214, 714) der Qualität jedes der Vielzahl von Teilsignalen (B₁, B₂, ..., B_{N-1}, B_{N}) das Berechnen einer Ähnlichkeit zwischen der repräsentativen Wellenform (41, 51, 61) und jedem Teilsignal (B₁, B₂, ..., B_{N-1}, B_{N}) oder einer Ähnlichkeit zwischen einem N-ten abgeleiteten Signal der repräsentativen Wellenform (41, 51, 61) und N-ten abgeleiteten Signalen jedes Teilsignals und das Bewerten der Qualität jedes Teilsignals (B₁, B₂, ..., B_{N-1}, B_{N}) basierend auf der berechneten Ähnlichkeit umfasst,
wobei das Bewerten der Qualität jedes der Vielzahl von Teilsignalen (B₁, B₂, ..., B_{N-1}, B_{N}) das Berechnen einer Ähnlichkeit zwischen der repräsentativen Wellenform (41, 51, 61) und jedem der Vielzahl von Teilsignalen (B₁, B₂, ..., B_{N-1}, B_{N}) während eines vorbestimmten Zeitintervalls vom Referenzpunkt (42a, 42b, 42c, 42d) jedes der Vielzahl von Teilsignalen (B₁, B₂, ..., B_{N-1}, B_{N}) umfasst.

8. Verfahren nach einem der Ansprüche 1 bis 7, wobei das Extrahieren (213, 713) der repräsentativen Wellenform (41, 51, 61) und das Bewerten (214, 714) der Qualität jedes der Vielzahl von Teilsignalen (B₁, B₂, ..., B_{N-1}, B_{N}) das wiederholte Bewerten der Qualität wenigstens eine vorbestimmte Zahl von Malen, und bis eine Ähnlichkeit zwischen einer repräsentativen Wellenform in einer aktuellen Iteration und einer repräsentativen Wellenform in einer vorhergehenden Iteration größer oder gleich einem ersten vorbestimmten Schwellenwert ist oder bis Qualitätsbewertungsergebnisse der Vielzahl von Teilsignalen in der aktuellen Iteration und Qualitätsbewertungsergebnisse der Vielzahl von Teilsignalen in der vorhergehenden Iteration größer oder gleich einem zweiten vorbestimmten Schwellenwert sind, umfassen.

9. Verfahren nach einem der Ansprüche 1 bis 8, wobei das Bewerten (215, 717) der Qualität des Signals im Qualitätsbewertungsbereich das Berechnen eines statistischen Wertes der Qualitätsbewertungsergebnisse jedes der Vielzahl von Teilsignalen und das Bewerten der Qualität des Signals im Qualitätsbewertungsbereich basierend auf dem berechneten statistischen Wert umfasst.

10. Vorrichtung zum Schätzen von Bioinformationen, wobei die Vorrichtung umfasst:
einen Sensor (110, 810), ausgebildet zum Messen eines Biosignals von einem Objekt; und
einen Prozessor (120, 820), ausgebildet zum:
Festlegen (211, 711) eines Qualitätsbewertungsbereichs im gemessenen Biosignal;
Unterteilen (212, 712) eines Signals des Qualitätsbewertungsbereichs in eine Vielzahl von Teilsignalen;
Extrahieren (213, 713) einer repräsentativen Wellenform (41, 51, 61) durch Verwenden der Vielzahl von unterteilten Teilsignalen (B₁, B₂, ..., B_{N-1}, B_{N});
Bewerten (214, 714) einer Qualität jedes der Vielzahl von Teilsignalen (B₁, B₂, ..., B_{N-1}, B_{N}) basierend auf der extrahierten repräsentativen Wellenform, um eine Qualität des Signals im Qualitätsbewertungsbereich zu bewerten (215, 717); und
Schätzen von Bioinformationen basierend auf der Qualitätsbewertung,
wobei der Prozessor (120, 820) ferner ausgebildet ist zum Erhalten (213, 713) der repräsentativen Wellenform (41, 51, 61) durch Anwenden von Gewichten (W₁, W₂, ..., W_{N-1}, W_{N}), die für die jeweiligen Teilsignale (B₁, B₂, ..., B_{N-1}, B_{N}) bestimmt sind, auf die jeweiligen Teilsignale (B₁, B₂, ..., B_{N-1}, B_{N}) und Extrahieren (213, 713) der repräsentativen Wellenform (41, 51, 61) durch Anpassen (716) der anzuwendenden Gewichte in einer aktuellen Iteration basierend auf Qualitätsbewertungsergebnissen von jeweiligen Teilsignalen in einer vorhergehenden Iteration.

11. Vorrichtung nach Anspruch 10, wobei der Prozessor (120, 820) ferner ausgebildet ist zum Ausführen von einem der folgenden Elemente:
Festlegen des Qualitätsbewertungsbereichs basierend auf Einheiten einer vorbestimmten Zahl von Herzschlägen oder eines vorbestimmten Zeitraums;
Unterteilen des Signals des Qualitätsbewertungsbereichs in die Vielzahl von Teilsignalen basierend auf Schlageinheiten;
Festlegen eines Referenzpunkts in jedem der Vielzahl von Teilsignalen und Extrahieren der repräsentativen Wellenform, die den Qualitätsbewertungsbereich darstellt, durch Überlappen der Vielzahl von Teilsignalen basierend auf dem festgelegten Referenzpunkt; und
Berechnen einer Ähnlichkeit zwischen der repräsentativen Wellenform und jedem der Vielzahl von Teilsignalen oder einer Ähnlichkeit zwischen einem N-ten abgeleiteten Signal der repräsentativen Wellenform und N-ten abgeleiteten Signalen jedes der Vielzahl von Teilsignalen und Bewerten der Qualität jedes der Vielzahl von Teilsignalen basierend auf der berechneten Ähnlichkeit.

12. Vorrichtung nach Anspruch 10 oder 11, wobei der Prozessor (120, 820) ferner zum wiederholten Bewerten der Qualität wenigstens eine vorbestimmte Zahl von Malen, und bis eine Ähnlichkeit zwischen einer repräsentativen Wellenform in einer aktuellen Iteration und einer repräsentativen Wellenform in einer vorhergehenden Iteration größer oder gleich einem ersten vorbestimmten Schwellenwert ist oder bis Qualitätsbewertungsergebnisse der Vielzahl von Teilsignalen in der aktuellen Iteration und Qualitätsbewertungsergebnisse der Vielzahl von Teilsignalen in der vorhergehenden Iteration größer oder gleich einem zweiten vorbestimmten Schwellenwert sind, ausgebildet ist.

13. Vorrichtung nach einem der Ansprüche 10 bis 12, wobei der Prozessor (120, 820) ferner zum Berechnen eines statistischen Wertes der Qualitätsbewertungsergebnisse jedes der Vielzahl von Teilsignalen und Bewerten der Qualität des Signals im Qualitätsbewertungsbereich basierend auf dem berechneten statistischen Wert ausgebildet ist.

14. Elektronische Vorrichtung, umfassend einen Hauptkörper und eine Vorrichtung zum Schätzen von Bioinformationen nach einem der Ansprüche 10 bis 13, die im Hauptkörper angeordnet ist.

15. Computerlesbares Speichermedium zum Speichern von Anweisungen, die, wenn von einem Prozessor (120, 820) ausgeführt, den Prozessor (120, 820) zum Ausführen des Verfahrens zum Bewerten der Qualität eines Biosignals veranlassen, umfassend die Schritte zum:
Empfangen eines gemessenen Biosignals;
Festlegen (211, 711) eines Qualitätsbewertungsbereichs im gemessenen Biosignal;
Unterteilen (212, 712) eines Signals des Qualitätsbewertungsbereichs in eine Vielzahl von Teilsignalen (B₁, B₂, ..., B_{N-1}, B_{N});
Extrahieren (213, 713) einer repräsentativen Wellenform (41, 51, 61) durch Verwenden der Vielzahl von unterteilten Teilsignalen (B₁, B₂, ..., B_{N-1}, B_{N});
Bewerten (214, 714) einer Qualität jedes Teilsignals (B₁, B₂, ..., B_{N-1}, B_{N}) basierend auf der extrahierten repräsentativen Wellenform;
Bewerten (215, 717) einer Qualität des Signals im Qualitätsbewertungsbereich basierend auf Qualitätsbewertungsergebnissen der Vielzahl von Teilsignalen; und
Schätzen von Bioinformationen basierend auf der Qualitätsbewertung,
wobei der Prozessor (120, 820) ferner angewiesen wird zum Erhalten (213, 713) der repräsentativen Wellenform (41, 51, 61) durch Anwenden von Gewichten (W₁, W₂, ..., W_{N-1}, W_{N}), die für die jeweiligen Teilsignale (B₁, B₂, ..., B_{N-1}, B_{N}) vorbestimmt sind, auf die jeweiligen Teilsignale (B₁, B₂, ..., B_{N-1}, B_{N}) und Extrahieren (213, 713) der repräsentativen Wellenform (41, 51, 61) durch Anpassen (716) der anzuwendenden Gewichte in einer aktuellen Iteration basierend auf Qualitätsbewertungsergebnissen von jeweiligen Teilsignalen in einer vorhergehenden Iteration.

## Revendications

1. Procédé implémenté par un ordinateur pour évaluer la qualité d'un bio-signal, le procédé comprenant :
la réception d'un bio-signal mesuré ;
la définition (211, 711) d'une région d'évaluation de qualité dans le bio-signal mesuré ;
la division (212, 712) d'un signal de la région d'évaluation de qualité en une pluralité de sous-signaux (B₁, B₂, ..., B_{N-1}, B_{N}) ;
l'extraction (213, 713) d'une forme d'onde représentative (41, 51, 61) en utilisant la pluralité de sous-signaux divisés (B₁, B₂, ..., B_{N-1}, B_{N}) ;
l'évaluation (214, 714) de la qualité de chaque sous-signal (B₁, B₂, ..., B_{N-1}, B_{N}) sur la base de la forme d'onde représentative extraite ;
l'évaluation (215, 717) de la qualité du signal dans la région d'évaluation de qualité sur la base des résultats d'évaluation de qualité de la pluralité de sous-signaux ;
et
l'estimation de la bio-information sur la base de l'évaluation de qualité,
dans lequel la forme d'onde représentative (41, 51, 61) est obtenue (213, 713) en appliquant des poids (W₁, W₂, ..., W_{N-1}, W_{N}), qui sont prédéterminés pour les sous-signaux respectifs (B₁, B₂, ..., B_{N-1}, B_{N}), aux sous-signaux respectifs (B₁, B₂, ..., B_{N-1}, B_{N}), et
dans lequel l'extraction (213, 713) de la forme d'onde représentative comprend en outre l'ajustement (716) des poids à appliquer dans une itération actuelle sur la base des résultats d'évaluation de qualité des sous-signaux respectifs dans une itération précédente.

2. Procédé selon la revendication 1, dans lequel la définition (211, 711) de la région d'évaluation de qualité comprend la définition de la région d'évaluation de qualité sur la base d'unités d'un nombre prédéterminé de battements de cœur ou d'une période prédéterminée.

3. Procédé selon la revendication 1 ou 2, dans lequel la division en une pluralité de sous-signaux comprend l'une des opérations suivantes :
division du signal de la région d'évaluation de qualité en sous-signaux respectifs sur la base d'unités de battement ;
définition d'un point de référence dans chaque sous-signal de la pluralité de sous-signaux ; et
dans lequel l'extraction de la forme d'onde représentative comprend l'extraction de la forme d'onde représentative, qui représente la région d'évaluation de qualité, en superposant la pluralité de sous-signaux sur la base du point de référence défini.

4. Procédé selon la revendication 3, dans lequel la définition du point de référence (42a, 42b, 42c, 42d) comprend la définition, comme point de référence, d'un point minimal (42a), d'un point maximal (42b), d'un point de pente maximale (42c) et d'un point d'intersection de tangente (42d) d'un sous-signal respectif.

5. Procédé selon la revendication 3, dans lequel l'extraction de la forme d'onde représentative comprend la superposition de la pluralité de sous-signaux après l'application d'un poids prédéfini à chaque sous-signal de la pluralité de sous-signaux.

6. Procédé selon la revendication 3, dans lequel l'extraction (213, 713) de la forme d'onde représentative comprend la superposition de la pluralité de sous-signaux après une normalisation de la pluralité de sous-signaux (B₁, B₂, ..., B_{N-1}, B_{N}) à une même taille.

7. Procédé selon l'une des revendications 1 à 6, dans lequel l'évaluation (214, 714) de la qualité de chaque sous-signal de la pluralité de sous-signaux (B₁, B₂, ..., B_{N-1}, B_{N}) comprend le calcul d'une similarité entre la forme d'onde représentative (41, 51, 61) et chaque sous-signal (B₁, B₂, ..., B_{N-1}, B_{N}), ou d'une similarité entre une n^{ième} dérivée de signal de la forme d'onde représentative (41, 51, 61) et une n^{ième} dérivée de signal de chaque sous-signal, et l'évaluation de la qualité de chaque sous-signal (B₁, B₂, ..., B_{N-1}, B_{N}) sur la base de la similarité calculée, dans lequel l'évaluation de la qualité de chaque sous-signal de la pluralité de sous-signaux (B₁, B₂, ..., B_{N-1}, B_{N}) comprend le calcul d'une similarité entre la forme d'onde représentative (41, 51, 61) et chaque sous-signal de la pluralité de sous-signaux (B₁, B₂, ..., B_{N-1}, B_{N}) durant un intervalle de temps prédéterminé à partir du point de référence (42a, 42b, 42c, 42d) de chaque sous-signal de la pluralité de sous-signaux (B₁, B₂, ..., B_{N-1}, B_{N}).

8. Procédé selon l'une des revendications 1 à 7, dans lequel l'extraction (213, 713) de la forme d'onde représentative (41, 51, 61) et l'évaluation (214, 714) de la qualité de chaque sous-signal de la pluralité de sous-signaux (B₁, B₂, ..., B_{N-1}, B_{N}) comprennent l'évaluation répétée de la qualité au moins un nombre prédéterminé de fois et jusqu'à ce qu'une similarité entre une forme d'onde représentative dans une itération actuelle et une forme d'onde représentative dans une itération précédente soit supérieure ou égale à une première valeur seuil prédéterminée, ou jusqu'à ce que les résultats d'évaluation de qualité de la pluralité de sous-signaux dans l'itération actuelle et les résultats d'évaluation de qualité de la pluralité de sous-signaux dans l'itération précédente soient supérieurs ou égaux à une deuxième valeur seuil prédéterminée.

9. Procédé selon l'une des revendications 1 à 8, dans lequel l'évaluation (215, 717) de la qualité du signal dans la région d'évaluation de qualité comprend le calcul d'une valeur statistique des résultats d'évaluation de qualité de chaque sous-signal de la pluralité de sous-signaux, et l'évaluation de la qualité du signal dans la région d'évaluation de qualité sur la base de la valeur statistique calculée.

10. Appareil d'estimation de bio-information, l'appareil comprenant :
un capteur (110, 810) configuré pour mesurer un bio-signal provenant d'un objet ; et
un processeur (120, 820) configuré pour :
définir (211, 711) une région d'évaluation de qualité dans le bio-signal mesuré ;
diviser (212, 712) un signal de la région d'évaluation de qualité en une pluralité de sous-signaux ;
extraire (213, 713) une forme d'onde représentative (41, 51, 61) en utilisant la pluralité de sous-signaux divisés (B₁, B₂, ..., B_{N-1}, B_{N}) ;
évaluer (214, 714) une qualité de chaque sous-signal de la pluralité de sous-signaux (B₁, B₂, ..., B_{N-1}, B_{N}) sur la base de la forme d'onde représentative extraite pour évaluer (215, 717) une qualité du signal dans la région d'évaluation de qualité ; et
estimer la bio-information sur la base de l'évaluation de qualité,
dans lequel le processeur (120, 820) est en outre configuré pour obtenir (213, 713) la forme d'onde représentative (41, 51, 61) en appliquant des poids (W₁, W₂, ..., W_{N-11} W_{N}), qui sont prédéterminés pour les sous-signaux respectifs (B₁, B₂, ..., B_{N-1}, B_{N}), aux sous-signaux respectifs (B₁, B₂, ..., B_{N-1}, B_{N}), et pour extraire (213, 713) la forme d'onde représentative (41, 51, 61) en ajustant (716) les poids à appliquer dans une itération actuelle sur la base des résultats d'évaluation de qualité de sous-signaux respectifs dans une itération précédente.

11. Appareil selon la revendication 10, dans lequel le processeur (120, 820) est en outre configuré pour effectuer l'une des opérations suivantes :
définition de la région d'évaluation de qualité sur la base des unités d'un nombre prédéterminé de battements de cœur ou d'une période prédéterminée ;
division du signal de la région d'évaluation de qualité en une pluralité de sous-signaux sur la base d'unités de battement ;
définition d'un point de référence dans chaque sous-signal de la pluralité de sous-signaux, et extraction de la forme d'onde représentative, qui représente la région d'évaluation de qualité, en superposant la pluralité de sous-signaux sur la base du point de référence défini ; et
calcul d'une similarité entre la forme d'onde représentative et chaque sous-signal de la pluralité de sous-signaux, ou d'une similarité entre une n^{ième} dérivée de signal de la forme d'onde représentative et une n^{ième} dérivée de signal de chaque sous-signal de la pluralité de sous-signaux, et évaluation de la qualité de chaque sous-signal de la pluralité de sous-signaux sur la base de la similarité calculée.

12. Appareil selon la revendication 10 ou 11, dans lequel le processeur (120, 820) est en outre configuré pour évaluer de manière répétée la qualité au moins un nombre prédéterminé de fois et jusqu'à ce qu'une similarité entre une forme d'onde représentative dans une itération actuelle et une forme d'onde représentative dans une itération précédente soit supérieure ou égale à une première valeur seuil prédéterminée, ou jusqu'à ce que les résultats d'évaluation de qualité de la pluralité de sous-signaux dans l'itération actuelle et les résultats d'évaluation de qualité de la pluralité de sous-signaux dans l'itération précédente soient supérieurs ou égaux à une deuxième valeur seuil prédéterminée.

13. Appareil selon l'une des revendications 10 à 12, dans lequel le processeur (120, 820) est en outre configuré pour calculer une valeur statistique des résultats d'évaluation de qualité de chaque sous-signal de la pluralité de sous-signaux, et pour évaluer la qualité du signal dans la région d'évaluation de qualité sur la base de la valeur statistique calculée.

14. Dispositif électronique comprenant un corps principal et un appareil d'estimation de bio-information selon l'une des revendications 10 à 13 qui est disposé dans le corps principal.

15. Support de stockage lisible par un ordinateur sur lequel sont stockées des instructions qui, quand elles sont exécutées par un processeur (120, 820), commandent au processeur (120, 820) d'exécuter le procédé d'évaluation de la qualité d'un bio-signal comprenant les étapes suivantes :
réception d'un bio-signal mesuré ;
définition (211, 711) d'une région d'évaluation de qualité dans le bio-signal mesuré ;
division (212, 712) d'un signal de la région d'évaluation de qualité en une pluralité de sous-signaux (B₁, B₂, ..., B_{N-1}, B_{N}) ;
extraction (213, 713) d'une forme d'onde représentative (41, 51, 61) en utilisant la pluralité de sous-signaux divisés (B₁, B₂, ..., B_{N-1}, B_{N}) ;
évaluation (214, 714) de la qualité de chaque sous-signal (B₁, B₂, ..., B_{N-1}, B_{N}) sur la base de la forme d'onde représentative extraite ;
évaluation (215, 717) de la qualité du signal dans la région d'évaluation de qualité sur la base des résultats d'évaluation de qualité de la pluralité de sous-signaux ;
et
estimation de la bio-information sur la base de l'évaluation de qualité,
dans lequel le processeur (120, 820) est en outre commandé pour obtenir (213, 713) la forme d'onde représentative (41, 51, 61) en appliquant des poids (W₁, W₂, ..., W_{N-11} W_{N}), qui sont prédéterminés pour les sous-signaux respectifs (B₁, B₂, ..., B_{N-1}, B_{N}), aux sous-signaux respectifs (B₁, B₂, ..., B_{N-1}, B_{N}), et pour extraire (213, 713) la forme d'onde représentative (41, 51, 61) en ajustant (716) les poids à appliquer dans une itération actuelle sur la base des résultats d'évaluation de qualité de sous-signaux respectifs dans une itération précédente.
